# EUROPEAN PATENT SPECIFICATION

(11) **EP 1 578 290 B1**
(45) Date of publication and mention of the grant of the patent: **28.03.2012**
(21) Application number: 03777029.4
(22) Date of filing: 24.11.2003
(51) Int. Cl.: A61B 19/00

(54) **APPARATUS AND METHOD FOR AUTOMATED POSITIONING OF A DEVICE**
GERÄT UND VERFAHREN FÜR DIE AUTOMATISCHE POSITIONIERUNG EINER VORRICHTUNG
APPAREIL ET PROCEDE POUR LE POSITIONNEMENT AUTOMATISE D'UN DISPOSITIF

(30) Priority: 06.12.2002 US 431452 P
(43) Date of publication of application: 28.09.2005
(73) Proprietor: Koninklijke Philips Electronics N.V., 5621 BA Eindhoven (NL)
(72) Inventor: TROVATO, Karen, I., Briarcliff Manor, NY 10510-8001 (US)
(74) Representative: Wolfs, Marc Johannes Maria
(86) International application number: PCT/IB2003/005428
(87) International publication number: WO 2004/052225

(56) References cited:
- WO-A-02/071315
- DE-A- 3 737 547
- US-A- 5 250 888
- US-B1- 6 181 983
- US-B1- 6 348 928

## Description

The present invention relates to an apparatus and method for positioning a device, such as a display screen.

Many devices, such as video monitors, instrument panels, protective barriers and display screens and other displays, are used in applications in which they must be kept in sight or remain conveniently accessible to a user. These devices may be used alone or as components of complex systems, such as medical imaging systems or machine tools. The user must often change position within a prescribed area while needing to keep the screen or other device in view.

Video monitors, in particular, often swivel or are located on stands which swivel or pivot or may be moved to a position using a scissor, yoke or other style support. A problem is that the user or other person must, typically, physically move the monitor or monitors. There may be a risk of repetitive stress injury (RSI) if the motion is frequent or the monitor or assembly containing one or more monitors, is heavy. There is, in any case, a considerable loss of efficiency.

Manual positioning and repositioning of monitors or other devices at an optimal position requires time, which the user may not have, or which may disrupt the activity underway. Not only may the inconvenience be considerable, but the risk to an operator, patient or others may be significant if, for example, both hands are required to position a screen, or if the operator must be in an inconvenient position or must divert his or her attention from other work, to move a video monitor or other device.

Thus, in many cases, a user's having to continually adjust the position of a device while using it, is not only inconvenient and inefficient, but can also pose a risk of injury to the user and others. Medical systems must often be operated using both hands. As the operator moves, the monitor or other device does not. Straining for an improved view may cause RSI, but may also increase the time required for a medical procedure.

Work related injuries could be reduced by the use of automated positioning of monitors. Clinical ultrasound, in particular, has ergonomic deficiencies. As many as 80% of sonographers report RSI's causing absence sometime during their careers. About 28% leave the practice due to RSI's. This not only is an immense human toll, but it further stresses the limited supply of sonographers so that longer hours and fewer breaks are often reported. The Society of Diagnostic Medical Sonography suggests "Ergonomically Designed Ultrasound Equipment," including an external monitor.

Current systems for automatic positioning have limited capabilities, particularly as to limitations on how the device is supported, range of movement and ability to adjust position and take obstacles into account, and are not very effective in addressing these problems. For example, two U.S. patents disclose systems providing limited tracking of a user and video monitors with limited movement. U.S. Patent no. 6,348,928 to Jeong discloses a system in which a visual display unit is capable of automatically rotating a display screen rightward or leftward following the viewer by detecting body temperature. U.S. Patent no. 6,311,141 to Hazra discloses a method and apparatus used with a display in which a physical relationship between the display and the viewer is determined and monitored and the display is rotated or moved on a table top to maintain the physical relationship.

One challenge to such systems is that the ideal position for the monitor or other device is often unobtainable, because of obstacles or other inherent limitations on the field of movement or view.

WO 02/071315 describes a system having a video display screen that provides video to a user. The position of the display screen is adjustable based upon the location of the user with respect to the display screen. The system includes at least one image capturing device trainable on a viewing region of the display screen and coupled to a control unit having image recognition software. The image recognition software identifies the user in an image generated by the image capturing device. The software of the control unit also generates at least one measurement of the position of the user based upon the detection of the user in the image.

It is, therefore, one object of the present invention to provide an apparatus and method of moving, without human effort or attention, a screen or other device to a desired, predetermined position.

Another object of the invention is to provide an apparatus and method of changing the desired position of a screen or other device as a user of the screen or other device moves.

These and still further objects of the present invention will become apparent upon considering the following detailed description for the present invention.

Accordingly, an apparatus and a method for automated positioning of a device according to the independent claims is provided.

In accordance with the invention, an achievable position nearest to an optimal position for use of a device is calculated, and, without effort or attention by a user, the device is positioned accordingly. Improved efficiency and ergonomics are provided because, among other reasons, user interaction is not required.

These objects are accomplished in one aspect of the invention by providing an apparatus comprising a sensor, which detects the presence and position of a subject, typically a user of a system, and transmits that information to a processor operatively connected to an arm assembly. The apparatus tracks the position of the user, particularly his or her face and/or eye locations so that the screen is automatically positioned to allow the user an optimal view. The screen position can be updated at time intervals or by defining a boundary of motion before an update occurs.

The sensor is typically a camera performing imaging using visible light. Infrared cameras, among other alternatives, can be used for the sensor, although the specific markers of additional heat can more readily identify two alternative users at a distance. The sensor may also be a transmitter which detects and relays information about the location and orientation of the user, by means of, for example, a array of electromagnetic coils. Multiple sensors may be used.

Data from the sensor is input to a processor subsystem which identifies the user's location and determines an optimal position and orientation for the device. The processor subsystem may be in a distributed computing environment.

The processor is a computer or computers, which calculates a location of the screen and the path the arm assembly will follow to move the screen to that location. This calculation is based on the capabilities of the actuators of the controlling machine of the arm assembly, size of the monitor and nearby forbidden regions such as walls, patient location, etc.

The mathematics of inverse kinematics can be used to find a vector of joint position variables satisfying the constraints of a given kinematic model of a mechanism and a set of position constraints on selected bodies making up that mechanism.

Typically, in moving to an achievable position nearest an optimal position, the apparatus will simply position the device at a "joint limit." In a more complex system, if, for example, the person's face is turned, then the system may compute position by first determining the position in space 2,54 CM*N or N inches (e.g. let N=18) from the center-of-eye position. The orientation of the eyes is next calculated. If the head is tipped, the eye-angle may be recorded. This defines the optimal location and orientation of the center of the device as well as the direction the device is to be facing. This optimal location (end-point) can be used as input directly to some robotic systems, or the inverse kinematics may be computed.

The arm assembly is a unit capable of structurally supporting and positioning a device in 3-dimensional space with 3-6 degrees of freedom. The device is connected to an end of the arm assembly. The arm assembly may be supported from a single point, such as a pole, footing or wall plate, and the arm assembly.

The arm assembly can comprise and be positioned using a controlling machine of, for example, motors such as servo or stepper motors. Commonly, these machines are positioned either by directing individual motor "setpoints," or by providing a location for an end effector, whereby the joint values are computed using inverse kinematics. Motors may be revolute or prismatic, which rotate about an axis, or linearly along an axis.

Degrees of freedom are independent parameters needed to specify a position of a body in a space. For example, the position in space of a hinged door can be set by one parameter, an opening angle. A hinged door thus has one degree of freedom. The position of a lawn mower operating on a flat patch of grass can be set by x- and y- position coordinates relative to x- and y- axes which are perpendicular to each other. A lawn mower on a flat surface thus has two degrees of freedom. The position and orientation of an object in three dimensional space can be set by specifying six degrees of freedom, three position coordinates and three orientation angles.

Robotic units capable of locating a device by specifying two to six degrees of freedom are commercially available and may advantageously be used as the arm assembly. Techniques for control and coordination of electro-mechanical machinery having multiple, interacting degrees of freedom are well known. For example, an arm manufactured by Unimation, Inc. under the tradename PUMA 560 can position an object in space at a location specified by six degrees of freedom.

The arm assembly may also have the ability to position a device with redundant degrees of freedom, i.e. degrees of freedom in excess of six, even though only six are necessary parameters for fixing the position and orientation of the device.

In one embodiment, the present invention is a system, which tracks the position of a user, particularly his or her face, neck and/or eye locations so that a screen automatically positions itself so that it is in front of a user, but giving the user the best possible view of a person or object, through the screen. The screen can be positioned to prevent, for example, the scatter of material such as blood or other fluids from a surgical procedure from reaching the operator. The screen may be a lens and thyroid protector, i.e. a plate of lead glass or other material, which absorbs radiation generated by a diagnostic or interventional x-ray procedure before it reaches the user. The screen position can be updated every N seconds, or by defining a boundary of motion before an update occurs.

An infrared or other proximity detector may be provided to detect an obstacle (such as another piece of equipment) or second person, in addition to the user, present near the arm or device. The detector can be interfaced with the controlling machine to prevent movement of the arm and device while the obstacle or second person is nearby.

These and other aspects of the invention will be apparent from and elucidated with reference to the embodiment(s) described hereinafter.

In the drawings:
FIG.1 1 shows an embodiment of a monitor stand according to the invention.
**FIG. 2** is a flowchart of a method of positioning the screen of the present invention.

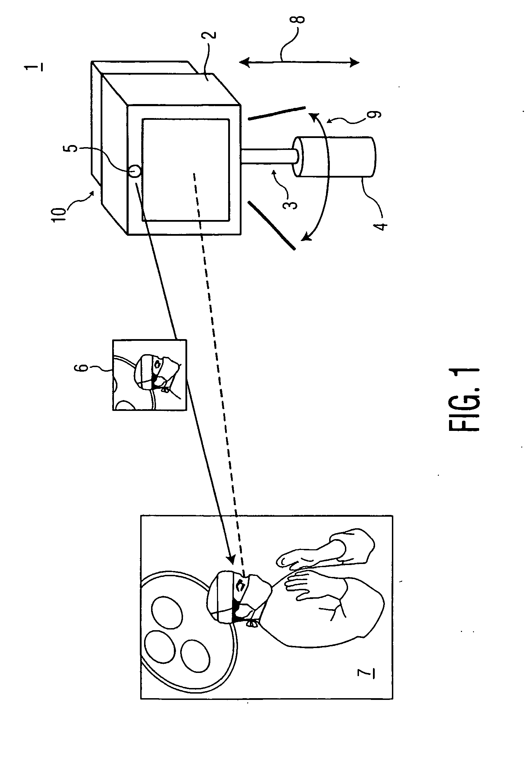

**FIG.1** shows a monitor stand **1** embodying the present invention. A monitor **2** is mounted on an arm assembly (here, a vertical arm) **3.** The arm assembly **3** is supported on a column **4.** A sensor **5** on the monitor **2** receives an image **6** of a user **7** and transmits the image data to a processor **10.**

The sensor **5** may be a camera, which is integrated into a monitor. The image **6** is detected, and face identified by the processor **10.** The monitor **2** has rotating motors or height-adjusting motors. A nominal distance such as 4,572 CM or 18 inches (about 0.5 meters) is often preferred for comfortable reading. The monitor is ideally positioned so that the user's eyes are centered relative to the screen. If the height is not adjustable, or the height is at the maximum, then the screen may be angled up or down to improve visibility. This then assumes that the monitor has additional degree of freedom.

In this simple example, the monitor **2** in a multi-user workspace may self-adjust to height only. The sensor **5** detects that the next person is, for example 198 CM (6'6") and the processor **10** determines that the ideal height adjustment **8** is to be 30,48 CM (1 foot) higher than the neutral position. It may, however, be that the possible range of motion is only 20,32 CM (+8 inches). In that case, the monitor will extend by 20,32 CM 8 inches, the nearest achievable position.

In a simple example, coordinates for predetermined positions may be stored. The recognition of the user can then simply set the device position at the location and orientation of the nearest predetermined position. In this case, no adaptive positioning occurs.

The monitor **2** may also have a greater range of motion in 3-dimensional space. In another case, the monitor **2** may even be 'held toward' the user, but back off as the user nears, allowing complete hands free operation of the screen. The optimal viewing pose for the monitor may comprise distance and orientation (typically 'straight ahead', zero degrees, 0.5 meters). Range of motion must be tested to ensure that the monitor has a limited range of motion, not affecting the workspace.

Although the sensor **5** is shown as a camera embedded in a monitor, this is not a requirement. The location of the sensor is also irrelevant as long as its performance is not disturbed by the monitor or other surrounding objects. For example, an RF transponder, as the sensor has different location requirements (e.g. sensitivity to radiofrequency interference or RFI) than a camera requiring line-of-sight.

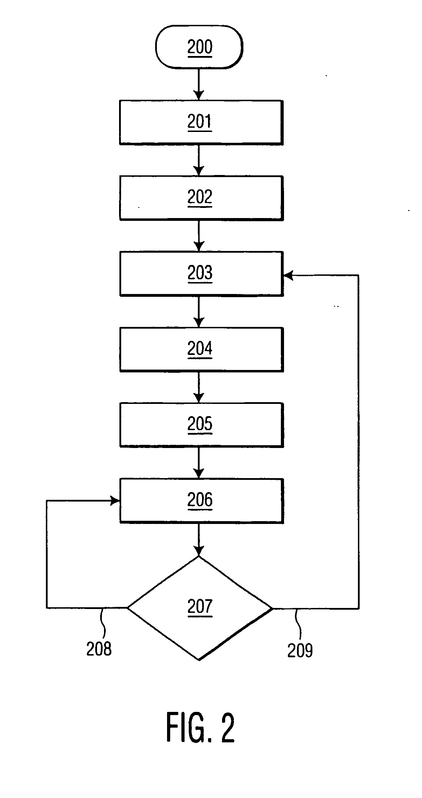

**FIG. 2** is a flowchart of a method of the present invention. In the embodiment of **FIG. 2****,** positioning a monitor or other screen using the system of the present invention comprises seven main steps. The process starts at **200.** A maximum window of allowable positions (which may include intermediate positions during motion), of the controlling joints that place the monitor are defined **201.** This window is sometimes called a work envelope or configuration space. The range of permissible joint angles in all combinations defines the window. They may be pre-defined, entered manually by a technician, or trained by moving the joints in combination and storing joint angles (such as from an encoder device). This calculation may include the position or limitations of the monitor or sensing device (e.g. camera), as well as any frequently anticipated machines in the local area.

The sensor is calibrated **202** with the location of the viewer or other user. In calibrating the sensor, the sensor data is processed using one or more algorithms to recognize and determine the coordinates of a person or object, as discussed below, to recognize a viewer and place the viewer by determining coordinates of the recognized viewer in 3-dimensional space. Ideally, for many embodiments of the invention, the viewer location is the position and orientation of the midpoint of the eyes. A distance and/or orientation offset from a location of a wearable sensor (e.g. RF transponder) may be used, or the viewer location may be calculated directly from the sensor (e.g. calculation of eye position from camera image).

For applications such as medical imaging systems, the comfortable monitor distance may be defined for each user. Further, it is important that the screen not move too frequently, which may also be defined for each user or type of situation.

The ideal viewing position for the monitor is calculated **203.** For example, a location 18 inches or 45 cm from the user, positioned with the top of the screen aligned with the center of the user's eyes may be considered optimal.

The achievable position nearest to the ideal viewing position is calculated **204.**

The screen is moved **205** to the achievable position using actuators of a controlling machine, for example, a robot. The robot will be limited to stay within the work envelope by the settings defined in step **201.**

The viewer location is calculated **206** and compared **207** to a repositioning criterion. Recalculation of the viewer location is directed **208** until the repositioning criterion is met **209.** For example; the criterion may be the viewer's having moved a distance (Δx, Δy, Δz) or rotated an angle (Δrx, Δry or Δrz) greater than a calculated threshold value. The repositioning criterion may also depend on a minimum or maximum amount of time having passed e.g. 5 seconds.

If the repositioning criterion is met **209,** the ideal viewing position based on the revised viewer location is calculated **203** and the steps **204, 205, 206** and **207** are repeated to set and maintain the new achievable position. The following example repositioning criterion establishes whether the user has moved substantially (for this application) and the monitor was not recently moved: Assuming that the user's mid-eye position is defined by x, y, z, if (Δx² + Δy² + Δ_{z}² > 6) and (time_since_last_movement > 10 seconds) then reposition_monitor.

If the location is outside the "reach" of the monitor, then the nearest point is found by tracing the eye-position through the monitor position to a location within the reachable locations. Ideally, the trace is the minimum distance. For multiple joint angles, the trace can be calculated by using the minimum distance in the "configuration space" of the arm and attached device, and simulated using a method, such as the path planning disclosed in U.S. patent no. 5,808,887, Animation of Path Planning, L. Dorst and K. Trovato.

Vision systems have been used to track objects. The cameras needed are currently inexpensive. There are many algorithms and techniques used to track objects from video sequences. For example, a detection and tracking module which extracts moving objects trajectories from a video stream is disclosed by G. Medioni et al., "Event Detection and Analysis from Video Streams," published by the University of Southern California Institute for Robotics and Intelligent Systems.

A gesture recognition system which locates face features in image frames is known from, for example, an article by J.B. Bishop et al. in "Automatic Head and Face Gesture Recognition," Technical Report no. FUTH TR001, published September 1, 2001 by Future of Technology and Health, LC, Iowa City, Iowa. A 3-D Face Recognition approach that is able to recognize faces in video sequences independent of face pose is disclosed by V. Krüger et al. in "Appearance-based 3-D Face Recognition from Video," University of Maryland Center for Automation Research, College Park, Maryland and The Robotics Institute, Carnegie Mellon University, Pittsburgh, Pennsylvania.

Yet another 3-D face recognition approach is a commercial product of Seeing Machines, Inc. of Canberra, Australia called "faceLAB™ V 1.1." This product can not only track head position, but also eye gaze, blinks and other, more subtle, behaviors.

A survey paper entitled "Object Detection and Tracking in Video", dated November, 2001, by Zhong Guo of the Department of Computer Science, Kent State University lists a number of approaches used for object detection and tracking, including deformable template matching and region based approaches to object tracking using motion information.

These methods can identify and provide an approximate location of an area of interest, including position and/or orientation of a pre-defined object such as a reflector, or even a person's face. There are also well-known stereoscopic and other techniques to determine the distance of an object from the camera. These methods typically analyze image geometry from views from two cameras. Image data from a single camera may be used. For example, Daphna Weinshall, Mi-Suen Lee, Tomas Brodsky, Miroslav Trajkovic and Doron Feldman, in an article entitled, "New View Generation with a Bi-centric Camera", Proceedings: 7th European Conference of Computer Vision, Copenhagen, May 2002, have proposed methods to extract 3D information from 2D video gathered from a single, uncalibrated camera.

Using only position (and not orientation), Tuttle in U.S. Pat. No 5,914,671 describes a system for locating an individual where a portable wireless transponder device is worn. Other radio (RF) techniques can be used to identify the position and orientation of a person or other object. Components which can compute the position and orientation of a small receiver as it moves through space, are commercially available. A system comprising a power supply, receiver, transmitter and hardware and software to generate and sense magnetic fields and compute position and orientation and interface with a host computer, is, for example, available under the name ISOTRAK II from Polhemus, Inc. of Colchester, Vermont. That system tracks six degrees of freedom in the movement of an object.

There are numerous ways, in addition to those mentioned above, to detect the location of an object. From that information, an estimate of the relative location of the person's eye midpoint may be calculated.

The devices to be positioned are not limited to video monitors, other display screens and protective shields.

The device may be a "cooperating device" that follows the movements of a user during a task, for example, a camera maintained in position with respect to a surgeon's hands or with respect to an instrument during surgery. The present invention may also, for instance, dynamically move speakers with respect to a listener's ears, or a keyboard with respect to the hands, or phone cradle and keys to match the height of a user.

The sensor may indicate that no user has been working with the system for N (e.g. 30) minutes, so that the device moves to a more neutral position, one more readily configured for the next user, or to a "rest" position out of the way of people who may be in the area.

The user has the ability to remove areas from the configuration space for the arm and device movement.

A cautionary note or symbol, e.g., a flashing border or notice on a display screen, may be displayed if the arm and device are in certain areas of the configuration space.

The processor may also monitor the user's position with respect to an object and provide an indication, warning notice or alarm if a user's position has changed in a way that might cause a display to confuse a user, in particular, if the user moves so that the orientation of the image displayed would appear to change.

"Comprising" does not exclude other elements or steps. "A" or "an" does not exclude a plurality. A single processor or other unit may fulfill the functions of several means recited in the claims.

## Claims

1. An apparatus (1) comprising:
a device (2);
an arm assembly (3) having a first end connected to a fixed support (4) and a second end connected to the device (2), said arm assembly (3) having actuating means for positioning the device;
wherein the arm assembly (3) is adapted for structurally supporting and positioning the device (2) in 3-dimensional space with 3-6 degrees of freedom;
a sensor (5) adapted to detect and provide information(6) about a subject (7) within a sensing range of the sensor (5);
a processor (10) to process said information (6), the processor (10) being configured to determine a location of the subject (7), determine a first location for the device and control the actuating means to move the arm assembly to position the device at a second location proximate to the first location, which second location is an achievable position of the arm assembly; and
a detector adapted for detecting an obstacle or a further person, said further person not being the subject,
wherein the movement of the arm assembly is prevented when said obstacle or further person is detected.

2. The apparatus of claim 1, wherein the first location and the second location are the same.

3. The apparatus of claim 1, wherein the processor (10) determines a path of the movement of the arm assembly to the second location using inverse kinematics.

4. The apparatus of claim 1, wherein the processor (10) determines a path of the movement of the arm assembly to the second location using path planning.

5. The apparatus of claim 1, wherein the processor (10) determines the second location using inverse kinematics.

6. The apparatus of claim 1, wherein the fixed support (4) is a single point.

7. The apparatus of claim 6, wherein the fixed support (4) is a pole.

8. The apparatus of claim 1, wherein the device (2) is a screen.

9. The apparatus of claim 8, wherein the screen is a shield.

10. The apparatus of claim 9, wherein the shield is a lens and thyroid protector.

11. The apparatus of claim 8, wherein the screen is a display screen.

12. The apparatus of claim 11, wherein the display screen is a video monitor.

13. The apparatus of claim 1, wherein the second location is chosen from two or more predetermined positions.

14. The apparatus of claim 1, wherein the processor (10) causes the actuating means of the arm assembly (3) to move the device to a rest position if the subject is not detected.

15. The apparatus of claim 1 further comprising a second sensor, the second sensor being configured to detect the presence of a person who is not the subject and being operatively coupled to the arm assembly (3) to prevent movement of the arm and the device if any said person who is not the subject is detected.

16. The apparatus of claim 1 wherein the first location is determined based on optimal viewing of the device.

17. The apparatus of claim 1 wherein the first location is determined based on optimal use of the device by the subject.

18. The apparatus of claim 1 wherein the first location is determined based on optimal viewing by the subject through the device.

19. The apparatus of claim 1 wherein the positions of the subject and device are monitored with respect to each other and a warning is displayed on a screen if certain changes in said positions are detected.

20. The apparatus of claim 1 wherein the positions of the subject and device are monitored with respect to each other and an alarm is activated if certain changes in said positions are detected.

21. A method for positioning a device comprising:
(a) calculating a window of allowable positions (201) ;
(b) calibrating a sensor with respect to a first user location (202) detecting and providing information (6) about a subject (7) within a sensing range of the sensor for
(c) calculating an ideal position (203) of the device based upon the first user location;
(d) calculating an achievable position within said window nearest the ideal position (204);
(e) moving the device within the window of allowable positions to the achievable position (205); and
(f) detecting an obstacle or a further person, said further person not being the subject; and
(g) preventing the movement of the arm assembly in case said obstacle or further person is detected.

22. The method of claim 21 further comprising: calculating (206) a second user location from sensor data collected after the first user location was determined; comparing the second user location with a repositioning criterion; repeating the step of calculating (206) a second user location from sensor data collected after the first user location was determined and comparing the second user location with the repositioning criterion, until the repositioning criterion is met (209); and repeating steps (c) and (d) of claim 21 to calculate a second ideal position of the device based on the second user location and a second achievable position; and moving the device within the window of allowable positions to the second achievable position (205).

## Patentansprüche

1. Gerät (1) mit:
einer Vorrichtung (2);
einer Armanordnung (3), die ein mit einem festen Träger (4) verbundenes erstes Ende und ein mit der Vorrichtung (2) verbundenes zweites Ende aufweist, wobei die Armanordnung (3) Betätigungsmittel zur Positionierung der Vorrichtung aufweist;
wobei die Armanordnung (3) so ausgeführt ist, dass sie die Vorrichtung (2) in einem dreidimensionalen Raum mit 3-6 Freiheitsgraden strukturell trägt und positioniert;
einem Sensor (5), der so eingerichtet ist, dass er Informationen (6) über ein Objekt (7) innerhalb eines Erfassungsbereichs des Sensors (5) detektiert und bereitstellt;
einem Prozessor (10) zur Verarbeitung der Informationen (6), wobei der Prozessor (10) so konfiguriert ist, dass er eine Position des Objekts (7) ermittelt, eine erste Position für die Vorrichtung ermittelt und die Betätigungsmittel so steuert, dass diese die Armanordnung so bewegen, dass die Vorrichtung in einer zweiten Position nahe der ersten Position positioniert wird, wobei es sich bei der zweiten Position um eine erreichbare Position der Armanordnung handelt, sowie
einem Detektor, der so eingerichtet ist, dass er ein Hindernis oder eine weitere Person detektiert, wobei es sich bei der weiteren Person nicht um das Objekt handelt,
wobei die Bewegung der Armanordnung verhindert wird, wenn das Hindernis oder die weitere Person detektiert wird.

2. Gerät nach Anspruch 1, wobei die erste Position und die zweite Position gleich sind.

3. Gerät nach Anspruch 1, wobei der Prozessor (10) unter Anwendung inverser Kinematik einen Weg der Bewegung der Armanordnung zu der zweiten Position ermittelt.

4. Gerät nach Anspruch 1, wobei der Prozessor (10) unter Anwendung von Path-Planning einen Weg der Bewegung der Armanordnung zu der zweiten Position ermittelt.

5. Gerät nach Anspruch 1, wobei der Prozessor (10) unter Anwendung inverser Kinematik die zweite Position ermittelt.

6. Gerät nach Anspruch 1, wobei es sich bei dem festen Träger (4) um eine einzelne Stelle handelt.

7. Gerät nach Anspruch 6, wobei der feste Träger (4) ein Pfosten ist.

8. Gerät nach Anspruch 1, wobei die Vorrichtung (2) ein Schirm ist.

9. Gerät nach Anspruch 8, wobei der Schirm eine Abschirmung ist.

10. Gerät nach Anspruch 9, wobei die Abschirmung eine Linse und ein Schilddrüsenprotektor ist.

11. Gerät nach Anspruch 8, wobei der Schirm ein Bildschirm ist.

12. Gerät nach Anspruch 11, wobei der Bildschirm ein Videomonitor ist.

13. Gerät nach Anspruch 1, wobei die zweite Position aus zwei oder mehreren vorgegebenen Positionen ausgewählt wird.

14. Gerät nach Anspruch 1, wobei der Prozessor (10) bewirkt, dass die Betätigungsmittel der Armanordnung (3) die Vorrichtung in eine Ruheposition bewegen, wenn das Objekt nicht detektiert wird.

15. Gerät nach Anspruch 1, das weiterhin einen zweiten Sensor umfasst, wobei der zweite Sensor so eingerichtet ist, dass er die Anwesenheit einer Person detektiert, bei der es sich nicht um das Objekt handelt, und mit der Armanordnung (3) betriebsbereit verbunden ist, um eine Bewegung der Armanordnung und der Vorrichtung zu verhindern, wenn eine solche Person, die nicht das Objekt ist, detektiert wird.

16. Gerät nach Anspruch 1, wobei die erste Position aufgrund optimaler Betrachtung der Vorrichtung ermittelt wird.

17. Gerät nach Anspruch 1, wobei die erste Position aufgrund optimaler Verwendung der Vorrichtung durch das Objekt ermittelt wird.

18. Gerät nach Anspruch 1, wobei die erste Position aufgrund optimaler Betrachtung durch das Objekt über die Vorrichtung ermittelt wird.

19. Gerät nach Anspruch 1, wobei die Position des Objekts und der Vorrichtung zueinander überwacht werden und eine Warnung auf einem Schirm angezeigt wird, wenn bestimmte Änderungen der Positionen detektiert werden.

20. Gerät nach Anspruch 1, wobei die Position des Objekts und der Vorrichtung zueinander überwacht werden und eine Warnung aktiviert wird, wenn bestimmte Änderungen der Positionen detektiert werden.

21. Verfahren zum Positionieren einer Vorrichtung, wobei das Verfahren die folgenden Schritte umfasst, wonach:
(a) ein Fenster zulässiger Positionen berechnet wird (201);
(b) ein Sensor im Hinblick auf eine erste Nutzerposition kalibriert wird, um Informationen (6) über ein Objekt (7) innerhalb eines Erfassungsbereichs des Sensors zu detektieren und bereitzustellen (202);
(c) eine ideale Position der Vorrichtung aufgrund der ersten Nutzerposition berechnet wird (203);
(d) eine der idealen Position am nächsten gelegene, erreichbare Position innerhalb des Fensters berechnet wird (204);
(e) die Vorrichtung innerhalb des Fensters zulässiger Positionen zu der erreichbaren Position bewegt wird (205); und
(f) ein Hindernis oder eine weitere Person detektiert wird, wobei die weitere Person nicht das Objekt ist; und
(g) die Bewegung der Armanordnung verhindert wird, im Falle das Hindernis oder die weitere Person detektiert wird.

22. Verfahren nach Anspruch 21, wonach weiterhin: eine zweite Nutzerposition anhand von Sensordaten, die nach Ermitteln der ersten Nutzerposition erfasst wurden, berechnet wird (206); die zweite Nutzerposition mit einem Repositionierungskriterium verglichen wird; der Schritt des Berechnens (206) einer zweiten Nutzerposition anhand von Sensordaten, die nach Ermitteln der ersten Nutzerposition erfasst wurden, wiederholt und die zweite Nutzerposition mit dem Repositionierungskriterium verglichen wird, bis das Repositionierungskriterium erfüllt wird (209); und Schritte (c) und (d) von Anspruch 21 wiederholt werden, um eine zweite ideale Position der Vorrichtung aufgrund der zweiten Nutzerposition und einer zweiten erreichbaren Position zu berechnen; und die Vorrichtung innerhalb des Fensters zulässiger Positionen zu der zweiten erreichbaren Position bewegt wird (205).

## Revendications

1. Appareil (1), comprenant :
un dispositif (2) ;
un ensemble bras (3) comportant une première extrémité reliée à un support fixe (4) et une seconde extrémité reliée au dispositif (2), ledit ensemble bras (3) comportant des moyens d'actionnement pour positionner le dispositif ;
dans lequel l'ensemble bras (3) est adapté pour supporter structurellement et positionner le dispositif (2) dans un espace tridimensionnel avec 3 à 6 degrés de liberté ;
un capteur (5) adapté pour détecter et fournir des informations (6) concernant un patient (7) au sein d'une portée de détection du capteur (5) ;
un processeur (10) pour traiter lesdites informations (6), le processeur (10) étant configuré pour déterminer une localisation du patient (7), déterminer une premier emplacement pour le dispositif et commander les moyens d'actionnement pour déplacer l'ensemble bras pour positionner le dispositif dans un second emplacement à proximité du premier emplacement, lequel second emplacement est une position pouvant être atteinte de l'ensemble bras ; et
un détecteur adapté pour détecter un obstacle ou une personne supplémentaire, ladite personne supplémentaire n'étant pas le patient,
dans lequel le mouvement de l'ensemble bras est empêché lorsque ledit obstacle ou ladite personne supplémentaire est détecté.

2. Appareil selon la revendication 1, dans lequel le premier emplacement et le second emplacement sont identiques.

3. Appareil selon la revendication 1, dans lequel le processeur (10) détermine un trajet du mouvement de l'ensemble bras jusqu'au second emplacement en utilisant une cinématique inverse.

4. Appareil selon la revendication 1, dans lequel le processeur (10) détermine un trajet du mouvement de l'ensemble bras jusqu'au second emplacement en utilisant une planification de trajet.

5. Appareil selon la revendication 1, dans lequel le processeur (10) détermine le second emplacement en utilisant une cinématique inverse.

6. Appareil selon la revendication 1, dans lequel le support fixe (4) est un point unique.

7. Appareil selon la revendication 6, dans lequel le support fixe (4) est un pilier.

8. Appareil selon la revendication 1, dans lequel le dispositif (2) est un écran.

9. Appareil selon la revendication 8, dans lequel l'écran est un dispositif de protection.

10. Appareil selon la revendication 9, dans lequel le dispositif de protection est une lentille et un cache thyroïde.

11. Appareil selon la revendication 8, dans lequel l'écran est un écran d'affichage.

12. Appareil selon la revendication 11, dans lequel l'écran d'affichage est un moniteur vidéo.

13. Appareil selon la revendication 1, dans lequel le second emplacement est choisi parmi deux, ou plus, positions prédéterminées.

14. Appareil selon la revendication 1, dans lequel le processeur (10) fait en sorte que les moyens d'actionnement de l'ensemble bras (3) déplacent le dispositif jusqu'à une position de repos si le patient n'est pas détecté.

15. Appareil selon la revendication 1, comprenant en outre un second capteur, le second capteur étant configuré pour détecter la présence d'une personne qui n'est pas le patient et étant accouplé de façon opérationnelle avec l'ensemble bras (3) pour empêcher le mouvement du bras et du dispositif si une quelconque personne qui n'est pas le patient est détectée.

16. Appareil selon la revendication 1, dans lequel le premier emplacement est déterminé en fonction d'une vision optimale du dispositif.

17. Appareil selon la revendication 1, dans lequel le premier emplacement est déterminé en fonction d'une utilisation optimale du dispositif par le patient.

18. Appareil selon la revendication 1, dans lequel le premier emplacement est déterminé en fonction d'une vision optimale par le patient à travers le dispositif.

19. Appareil selon la revendication 1, dans lequel les positions du patient et du dispositif sont surveillées l'une par rapport à l'autre et un avertissement est affiché sur un écran si certains changements desdites positions sont détectés.

20. Appareil selon la revendication 1, dans lequel les positions du patient et du dispositif sont surveillées l'une par rapport à l'autre et une alarme est activée si certains changements desdites positions sont détectés.

21. Procédé pour positionner un dispositif, comprenant les étapes consistant à :
(a) calculer une fenêtre de positions admissibles (201) ;
(b) calibrer un capteur par rapport à un premier emplacement d'utilisateur (202) pour détecter et fournir des informations (6) concernant un patient (7) au sein d'une portée de détection du capteur ;
(c) calculer une position idéale (203) du dispositif en fonction du premier emplacement d'utilisateur ;
(d) calculer une position pouvant être atteinte au sein de ladite fenêtre la plus proche de la position idéale (204) ;
(e) déplacer le dispositif au sein de la fenêtre de positions admissibles jusqu'à la position pouvant être atteinte (205) ; et
(f) détecter un obstacle ou une personne supplémentaire, ladite personne supplémentaire n'étant pas le patient ; et
(g) empêcher le mouvement de l'ensemble bras au cas où ledit obstacle ou ladite personne supplémentaire est détecté.

22. Procédé selon la revendication 21, comprenant en outre les étapes consistant à : calculer (206) un second emplacement d'utilisateur à partir de données de capteur collectées après que le premier emplacement d'utilisateur a été déterminé ; comparer le second emplacement d'utilisateur à un critère de repositionnement ; répéter l'étape consistant à calculer (206) un second emplacement d'utilisateur à partir de données de capteur collectées après que le premier emplacement d'utilisateur a été déterminé et comparer le second emplacement d'utilisateur au critère de repositionnement, jusqu'à ce que le critère de repositionnement soit satisfait (209) ; et répéter les étapes (c) et (d) selon la revendication 21 pour calculer une seconde position idéale du dispositif en fonction du second emplacement d'utilisateur et une seconde position pouvant être atteinte ; et déplacer le dispositif au sein de la fenêtre de positions admissibles jusqu'à la seconde position pouvant être atteinte (205).
